(19) Europäisches Patentamt
European Patent Office
Office européen des brevets

(11) Publication number: **0 489 219 A1**

(12) # EUROPEAN PATENT APPLICATION

(21) Application number: **91100266.5**

(51) Int. Cl.$^5$: **C09K 11/07**, F21K 2/06

(22) Date of filing: **10.01.91**

(30) Priority: **02.12.90 US 478308**

(43) Date of publication of application:
**10.06.92 Bulletin 92/24**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Walter, Smithey A.**
**671 Martin Street**
**Pace, Florida 32571(US)**

(74) Representative: **Wächtershäuser, Günter, Dr.**
**Tal 29**
**W-8000 München 2(DE)**

(54) **Visible light reduction in non-visible chemical light devices.**

(57) The tendency for non-visible light generating chemical light devices to generate undesirable amounts of visible light is obviated by the inclusion of a visible-light absorbing, or wavelength shifting material.

EP 0 489 219 A1

## Background Of The Invention

U.S. Patent 4,379,320 discloses and claims a chemical light device of the type comprising a liquid chemiluminescent mixture in a transparent or translucent container. These devices are of the kind comprising an outer flexible sealed compartment containing components of a chemiluminescent mixture each separated by frangible means inside the compartment such as a glass or plastic ampule. By bending the compartment, the frangible means is fractured and the components of the chemiluminescent mixture are allowed to mix to thereby form light which is emitted through the container walls.

Typically, a chemiluminescent mixture comprises a chemiluminescent compound such as an oxalate diester which reacts with a peroxide component, usually hydrogen peroxide, and a fluorescer which is activated by the chemiluminescent reaction. Catalysts or activators may also be included, usually combined with the peroxide, and all the components are usually present in liquid form dissolved in a solvent.

One type of chemical light device which has been proven to be very effective, especially for military uses, is that which contains a fluorescer which emits in the IR region of the spectrum, i.e, at a wavelength of 700 nanometers or longer. These devices are useful as markers in areas where it is desired that detection go unnoticed i.e. areas where infiltration is to be kept at a minimum. These markers, because they emit only in the IR region, can only be detected via the use of infrared detecting instruments.

One of the problems that has continually faced the industry is the tendency of infrared light devices to generate detectable amounts of visible light thereby detracting from the efficacy of the device. The visible light emission is generally due to the presence of trace amounts of impurities which may comprise derivatives of the IR fluorescer or other components used in the production of the IR fluorescer. Previously, elimination of the visible light was attempted by either continually purifying the fluorescer such as by a series of recrystallizations etc. to remove the impurities or by the use of high concentrations of the IR fluorescer in the chemical light device whereby the visible light was self-absorbed. Both of these methods of reducing the visible light, besides being very expensive, failed to successfully solve the problem, in that, the use of high concentrations of fluorescer and purification never completely resulted in total visible light removal, and, at high concentrations, the IR fluorescer, but not the visible light emitting impurities, had a tendency to precipitate out of solution after a period of time and thereby create an even greater visible light problem.

Accordingly, there remains a need for chemical light devices which 1) emit in the IR range, 2) are free from any visible light emission and 3) can be produced at a price such as to render the device viably commercial. Satisfaction of this long-felt need would provide a worthy public benefit in a very important field.

## Summary of the Invention

The present invention relates to a chemical light device comprising a chemiluminescent mixture containing an IR fluorescer inside a container, and, associated therewith, a material capable of absorbing visible light emitted by the chemiluminescent mixture or shifting the visible light to IR wavelengths by dispersion.

## Description of the Invention Including Preferred Embodiments

The present invention relates to a chemical light device comprising 1) a chemiluminescent mixture containing an IR fluorescer activated by the reaction of a chemiluminescent compound with a peroxide component, all contained inside a transparent or translucent container, and 2) a material capable of absorbing or shifting the wavelength of visible light emitted by the IR fluorescer mixture associated therewith.

The novel devices of this invention comprise a container for a chemiluminescent mixture with a material which absorbs visible light or shifts the wavelength thereof to IR associated therewith. The material can be a) incorporated into the walls of the container, b) incorporated into a polymer sleeve surrounding the exterior or positioned at the interior of the container, c) coated onto the interior walls or the exterior walls of the container or d) dispersed or dissolved in the chemiluminescent liquid contents of the container. It is preferred that the material be incorporated into the walls of the container.

The visible light absorbing or wavelength shifting material must not only absorb or shift the visible light wavelength but allow the passage of the IR light emitted by the IR fluorescer mixture, preferably to the substantial exclusion of any IR light absorption. Useful visible light absorbing or wavelength shifting materials include dyes, pigments etc. which may or not be fluorescers themselves. They may be organic or

inorganic. It is preferred that the visible light absorbers or wavelength shifters not be visible light emitting fluorescers, i.e. if they are fluorescers they must not be capable of excitation such as to emit light into the visible spectrum either by light emitted from the IR fluorescer or otherwise. It is even more preferred that the visible light absorbers or wavelength shifters be capable of emittting IR upon being stimulated by visible light.

Examples of suitable visible light absorbers and wavelength shifters include nigrosines, oil-soluble azos, diazos, anthraquinones, pyrazolones, quinophthalones, quirolines, phthaloperinones, cadmium pigments, iron oxides, cobalt aluminates, black spinels, chromium oxide green, ferric ammonium ferrocyanide, ultramarine blue, phthalocyanine, nickel azo yellow, carbazole violet, carbon black, pearlescent pigments, metallic flake pigments, daylight fluorescent pigments, color concentrates and the like and mixtures thereof.

Any solid, visible light absorbing or wavelength shifting materials employed herein should preferably have a particle size larger than about 2 microns in diameter.

Examples of materials which not only absorb visible light but emit IR light upon stimulation include zinc sulfide, zinc selenide and zinc, germanate glass, impure germanium, n-type silicon, $S_1O_2$ coated silicon, arsenic doped silicon etc, see Applied Optics, Vol. 5, No. 12, pages 1899-1901 December 1966, hereby incorporated herein by reference.

The amount of visible light absorbing or wavelength shifting material employed ranges from about .5 to about 10%, by weight, based on the weight of the polymer or solution into which it is dispersed or dissolved or onto which it is coated, preferably from about 1.0 to about 7.5%, by weight, same basis. The material may be incorporated into the container walls, which are preferably a transparent or translucent plastic material, and most preferably a polyolefin such as polyethylene, polypropylene etc, by known procedures such a dry blending the material and the plastic in powder or granular form etc., followed by milling, Banbury mixing, molding, casting, extruding etc. to obtain a flexible container such as a wrap, sleeve, tube, bubble etc. The thickness of the container wall governs the concentration of visible light absorbing or wavelength shifting material incorporated therein, the thicker the wall, the less the concentration of material needed i.e. one must maintain a desired optical density in the visible spectral region. When the visible light absorbing or wavelength shifting material is positioned in a sleeve-like device, it can be fitted over or into the compartment containing the IR fluorescer containing chemiluminescent mixture as long as the sleeve covers the entire surface area of the container. If in the form of a wrapping, the wrapping may be wrapped against the outer wall of the container in one or more layers.

Since visible light in IR devices varies inversely with the concentration of the IR fluorescer, the concentration of the visible light absorbing or wavelength shifting material used must be adjusted accordingly, i.e. as the concentration of the IR fluorescer decreases, the amount of visible light emitted increases. At levels of IR fluorescer over about 0.3% in the oxalate component, very low visible light absorbing or wavelength shifting material concentrations are necessary, e.g. about 0.5% in a 50 nil thick wall. At low levels of IR absorber (0.5-2.0%), about 2% visible light absorbing or wavelength shifting material in a 50 mil wall is needed.

Chemilumenescent light devices are well known in the art, see for example U.S. 3,539,794; 3,729,425, 3,893,938, and 4,814,949, all incorporated herein by reference. Generally, these devices all function similarly in that they are comprised of compartments containing glass ampules, for example, which separate mixtures of the chemiluminescent components from one another before activation of the device. As mentioned above, one mixture usually comprises a solvent solution of the oxalate and IR fluorescer and the other mixture usually comprises a solvent solution of the peroxide and catalyst. Suitable solvents include dibutyl phthalate or butyl benzoate alone or in admixture with t-butyl alcohol. Any IR fluorescer may be used with those set forth in U.S. Patent No. 3,630,941 incorporated herein by reference, being exemplary.

These IR fluorescers have the formula:

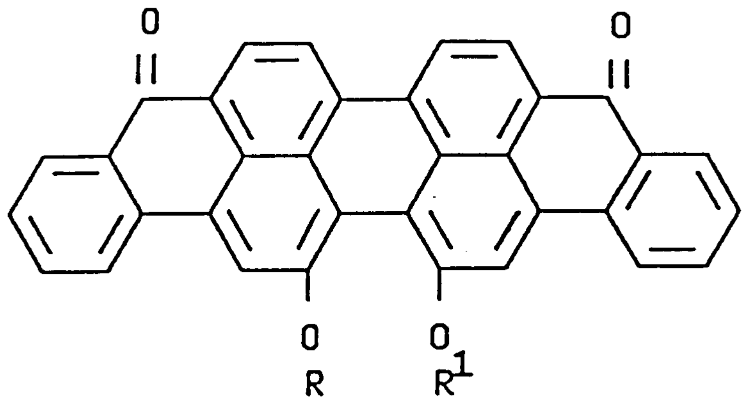

wherein R and $R^1$, individually, are alkyl groups of 1-18 carbon atoms, inclusive.

Additionally, such IR fluorescers such as diethyloxatricarbocyanine may be used.

Chemiluminescent chemicals disclosed in the above-referenced patents are useful herein.

The following examples are set forth for purposes of illustration only and are not to be construed as limitations on the present invention except as set forth in the appended claims. All parts and percentages are by weight unless otherwise specified.

In the following examples, all testing is conducted on IR light devices comprising 6 inch long tubes. In those cases where the tube contains a visible light absorbing or wavelength shifting material in the wall thereof, the material is added to plastic (polyethylene) pellets, tumbled to mix and extruded into molds. The term "standard", when used herein, means the component of corresponding commercially available chemical light device chemiluminescent mixtures. The IR fluorescer in each instance is 16,17-didecyloxyviolanthrone (DDV).

Example 1

A standard oxalate component of a standard IR emitting chemical light device containing 0.377% DDV is diluted with fluorescer-free oxalate solution to result in 50%, 40%, 30%, and 20% sample solutions of the standard concentration. The resultant oxalate solutions are placed into a) clear 6" polyethylene tubing with one sealed end and b) like sizes of the same tubing containing 2 weight percent of Radiant K 600 39 fluorescent blue pigment dispersed in the plastic.

In addition, the undiluted (100%) oxalate solution is also so treated. Each device is activated with a standard peroxide solution and the visible light is then measured one (1) minute after activation. The results are set forth in Table I, below. The visible light from the clear tubing is red and the visible light from the blue dyed tubing is bluish-white.

Table I

| DDV Concentration (% of Standard) | Visible Light (Luminance-Foot Lamberts X10-$^{2}$) (Two Samples each) | |
|---|---|---|
| | Clear Tubing | Blue Tubing |
| 100 | .04<br>.05 | .00<br>.00 |
| 50 | .16<br>.15 | .00<br>.00 |
| 40 | .20<br>.21 | .00<br>.00 |
| 30 | .24<br>.27 | .01<br>.00 |
| 20 | .45<br>.50 | .03<br>.02 |

It is evident that the presence of the blue dye in the wall of the polyethylene tubing effectively and efficiently prevents the escape of visible light emitting from the device. Note also that reduction of the IR fluorescer concentration to 40% of the standard also is achieved by the presence of the blue dye, a further advantage of the instant devices. In each instance, the total IR light is substantially equal.

Example 2

A set of polyethylene casings is prepared from compounded polymer. Colorant is added to the polymer resin, mixed and extruded into pellets. The pellets are again extruded and then molded into the casings. The casings are very uniform in color. The same dye as in Example 1 is employed. Standard oxalate solution containing .128% DDV is added to the casings after sealing one end thereof and activated with standard peroxide solution. Visible light one minute after activation and five minutes after activation is measured using a Spectra Spot Meter - Model UB 1/4 equipped with phototropic filter. The results are set forth in Table II, below.

Table II

| | Visible Light (Luminance-Foot Lamberts X10-$^{2}$) (Two Samples each) | |
|---|---|---|
| | Clear Casing | Blue Casing |
| Activation plus 1 minute<br>Activation plus 5 minutes | .21<br>.13 | .01<br>.00 |

All casings meet irradiance specifications. Only the blue casings meet visible light specifications.

Example 3

The procedure of Example 1 is again followed except that the devices tested are produced in accordance with U.S. Patent No. 4,814,949. The devices are round in shape. One device is formed from a blue pigmented polypropylene sheet containing a mixture of three (3) pigments to a concentration of about 15% and a second device is formed from a clear polypropylene sheet. Upon activation, the device produced from the clear polypropylene sheet exhibits excessive visible light which is very visible to the dark-adapted eye. The visible light is tested at 0.09 x $10^{-2}$ Foot Lamberts. The device produced from the blue pigmented polypropylene sheet is tested at less than 0.001 x $10^{-2}$ Foot Lamberts.

Examples 4-13

Following the procedure of Example 1, various pigments and dyes are substituted for the blue pigment thereof. The results are substantially the same as achieved in Example 1.

| | |
|---|---|
| 4. Dayglo blue. | 10. Chromium oxide green. |
| 5. Phthalo blue. (A) | 11. Nickel azo yellow. |
| 6. Ultramarine blue. (A) | 12. Carbazole violet. (A) |
| 7. Cobalt blue. | 13. Bismuth oxychloride. |
| 8. Carbon black (2M*). | 14. Zinc sulfide. ** |
| 9. Ca/Hg sulfide solid sol. | 15. Zinc Selenide and Zinc. ** |

*particles -2 microns in diameter-dispersed in the oxalate component of the chemiluminescent mixture.
(A) = dissolved in oxalate component of chemiluminescent mixutre.
**IR emission of device enhanced over use of IR fluorescer DDV alone.

## Claims

1. A chemical light device comprising 1) a chemiluminescent mixture containing an IR fluorescer activated by the reaction of a chemilumenescent component with a peroxide component, all contained inside a transparent or translucent container and 2) a material capable of absorbing or shifting the wavelength of visible light emitted by the IR fluorescer mixture associated with said container.

2. A chemical light device according to Claim 1 wherein the visible light absorbing or wavelength shifting material is dispersed in a plastic constituting the walls of said container containing said chemiluminescent mixture.

3. A chemical light device according to Claim 1 wherein said visible light absorbing or wavelength shifting material is dispersed in a plastic sleeve surrounding the exterior of said container which contains said chemiluminescent mixture.

4. A chemical light device according to Claim 1 wherein said IR fluorescer is a 16,17-dialkoxyviolanthrone.

5. A chemical light device according to Claim 4 wherein said IR fluorescer has the formula:

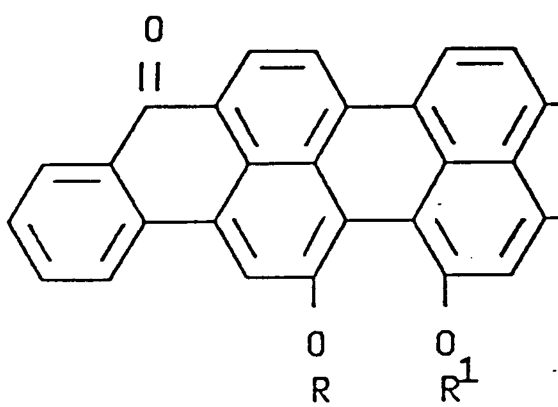

wherein R and $R^1$ are, individually, alkyl groups of 1-18 carbon atoms, inclusive.

6. A chemical light device according to Claim 5 wherein both R and $R^1$ are decyl.

7. A chemical light device according to Claim 5 wherein both R and $R^1$ are hexyl.

8. A chemical light device according to Claim 1 wherein said visible light absorbing or wavelength shifting material is a blue dye.

9. A chemical light device according to Claim 1 in the shape of a tube.

10. A chemical light device according to Claim 1 wherein said visible light absorbing or wavelength shifting material is a non-visible fluorescer.

11. A chemical light device according to Claim 1 wherein said visible light absorbing or wavelength shifting material emits IR light.

12. A chemical light device according to Claim 1 wherein said visible light absorbing or wavelength shifting material is present as a dispersion in said chemiluminescent mixture.

13. A chemical light device according to Claim 12 wherein said visible light or wavelength shifting material is present as dispersed particles.

14. A chemical light device according to Claim 12 wherein said visible light absorbing or wavelength shifting material is present as a dissolved dye or pigment.

European Patent Office

# EUROPEAN SEARCH REPORT

Application Number

EP 91 10 0266

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-3 630 941 (W.R. BERGMARK) * Column 1, lines 18-23; column 1, line 53 - column 2, line 28; column 2, lines 37-52; claims * --- | 1,4,5,7 | C 09 K 11/07<br>F 21 K 2/06 |
| D,A | EP-A-0 061 558 (AMERICAN CYANAMID CO.) * Abstract; page 1, line 28 - page 3, line 18; page 5, line 13 - page 6, line 9; claims 1-3 * ----- | 1,2,3,9 | |

TECHNICAL FIELDS SEARCHED (Int. Cl.5)

C 09 K

The present search report has been drawn up for all claims

| Place of search | Date of completion of the search | Examiner |
|---|---|---|
| THE HAGUE | 28-06-1991 | VAN DER POEL W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)